(19) Europäisches Patentamt
European Patent Office
Office européen des brevets

(11) Veröffentlichungsnummer: **0 213 079 A2**

# (12) EUROPÄISCHE PATENTANMELDUNG

(21) Anmeldenummer: **86810366.4**

(22) Anmeldetag: **18.08.86**

(51) Int. Cl.⁴: **C 07 D 417/14**, C 07 D 409/14, C 07 D 417/12, C 07 D 409/12

(30) Priorität: **23.08.85 CH 3643/85**
**28.02.86 CH 811/86**

(43) Veröffentlichungstag der Anmeldung: **04.03.87**
**Patentblatt 87/10**

(84) Benannte Vertragsstaaten: **BE CH DE FR GB IT LI NL**

(71) Anmelder: **CIBA-GEIGY AG, Klybeckstrasse 141, CH-4002 Basel (CH)**

(72) Erfinder: **Dürr, Dieter, Dr., Brändelistalweg 16, CH-4103 Bottmingen (CH)**
Erfinder: **Szczepanski, Henry, Dr., Bodenmatt, CH-4323 Wallbach (CH)**

## (54) Thiazolylalkyl- und Thienylalkylester von alpha-Imidazolinon-nicotin- und -benzoesäuren.

(57) Neue Thiazolalkyl- und Thienylalkylester von α-Imidazolinon-nicotin- und -benzoesäuren der untenstehenden Formel I haben gute pre- und postemergente selektiv-herbizide Eigenschaften, ferner beeinflussen, respektive hemmen sie das Pflanzenwachstum.

Die neuen Ester entsprechen der Formel I

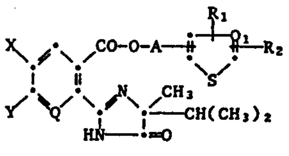

(I)

worin

A ein geradkettiges oder verzweigtes $C_1$-$C_6$-Alkylenbrückenglied,

Q und $Q_1$ unabhängig voneinander je Stickstoff oder die Methingruppe,

$R_1$ und $R_2$ unabhängig voneinander je Wasserstoff oder $C_1$-$C_4$-Alkyl,

X und Y unabhängig voneinander je Wasserstoff, $C_1$-$C_4$-Alkyl, $C_1$-$C_4$-Alkoxy, $C_3$-$C_4$-Alkenyl, $C_1$-$C_4$-Halogenalkyl, Halogen oder zusammen den Butadienrest bedeuten.

EP 0 213 079 A2

0213079

CIBA-GEIGY AG
Basel (Schweiz)

5-15476/1+2/=

Thiazolylalkyl- und Thienylalkylester von α-Imidazolinon-nicotin- und -benzoesäuren

Die vorliegende Erfindung betrifft neue Thiazolylalkyl- und Thienylalkylester von α-Imidazolinon-nicotin- und -benzoesäuren mit herbizider und den Pflanzenwuchs regulierender Wirkung, sowie die Herstellung dieser neuen Verbindungen. Ferner betrifft sie Mittel, welche die neuen Imidazolinon-Verbindungen enthalten sowie deren Verwendung zur selektiven Bekämpfung von Unkräutern oder zur Regulierung des Pflanzenwuchses.

Die neuen Thiazolylalkyl-, Thienylalkylester von α-Imidazolinon-nicotin- und -benzoesäuren entsprechen der Formel I

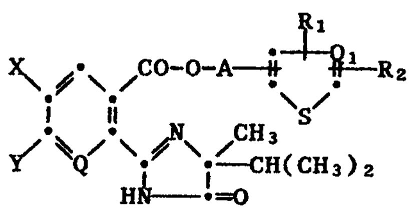

(I)

worin

A eine geradkettige oder verzweigte $C_1$-$C_6$-Alkylenbrücke,
Q und $Q_1$ unabhängig voneinander je Stickstoff oder die Methingruppe,
$R_1$ und $R_2$ unabhängig voneinander je Wasserstoff oder $C_1$-$C_4$-Alkyl,
X und Y unabhängig voneinander je Wasserstoff, $C_1$-$C_4$-Alkyl, $C_1$-$C_4$-Alkoxy, $C_3$-$C_4$-Alkenyl, $C_1$-$C_4$-Halogenalkyl, Halogen oder zusammen den Butadienrest bedeuten.

In diesen Definitionen können die Alkylreste, wie auch die Alkylenreste, geradkettig oder verzweigt sein und die Bedeutung von Methyl, Methylen, Aethyl, Aethylen, Propyl, Propylen, Isopropyl, 1- oder 2-Methyläthylen, Butyl, Butylen, sec. Butyl, 1-Methylpropylen, Isobutyl, 2-Methylpropylen, tert.-Butyl, 2,2-Dimethyläthylen, 1,2-Dimethyläthylen, haben.

Halogen bedeutet Fluor, Chlor, Brom oder Iod.

Die Herstellung solcher Verbindungen lässt sich durch folgendes Schema darstellen:

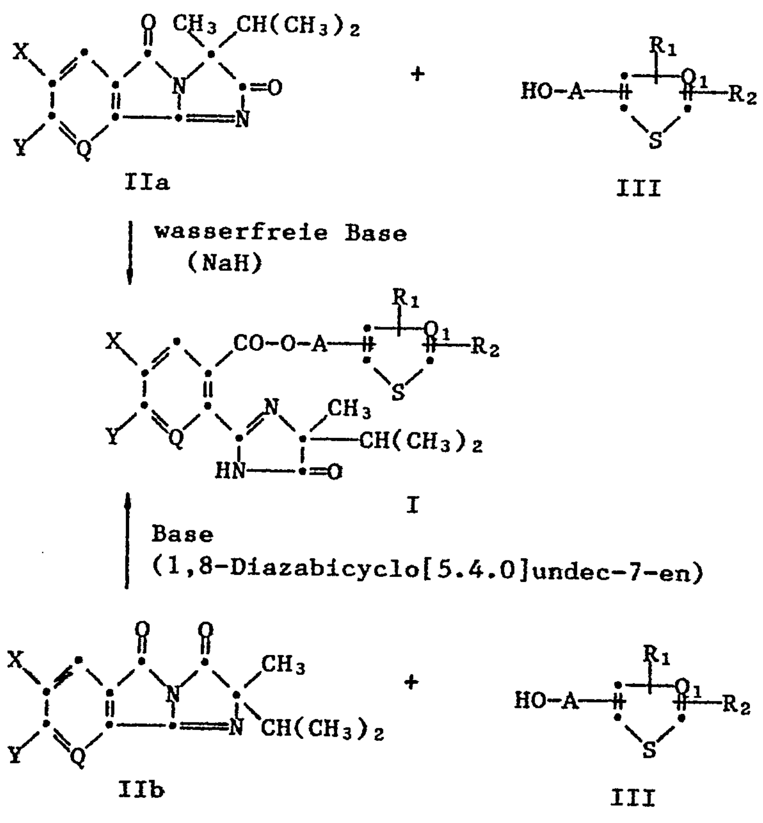


IIa III

I

IIb III

Das erfindungsgemässe Verfahren zur Herstellung der Imidazolinon-Verbindungen der Formel I besteht darin, dass man eine Verbindung der Formel IIa resp. IIb

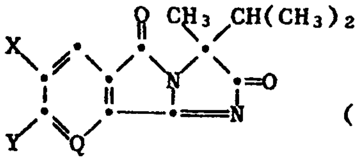 (IIa) 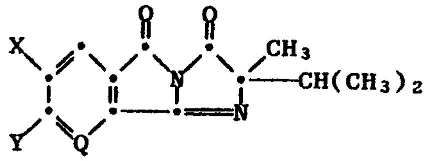 (IIb)

worin Q, X und Y die unter der Formel I gegebene Bedeutung haben, in einem inerten organischen Lösungs- oder Verdünnungsmittel, in Gegenwart eines basischen Mittels mit einem Thiazolylalkohol oder Thienylalkohol der Formel III umsetzt,

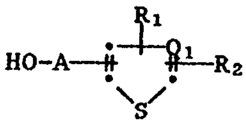 (III)

worin A, $Q_1$, $R_1$ und $R_2$ die unter der Formel I angegebene Bedeutung haben.

Als Lösungsmittel für diese Umsetzungen eignen sich möglichst wasserfreie Kohlenwasserstoffe, Aether oder Ketone, wie z.B. Benzol, Toluol, Xylol, Hexan, Cyclohexan, Diisopropyläther, Tetrahydrofuran, Dioxan.

Als basische Mittel kommen z.B. Natriumhydrid, 1,8-Diaza-bicyclo-[5.4.0]undec-7-en, tert. Amine und Alkalihydroxide in Frage.

Diese Reaktionen werden bei Temperaturen zwischen 0 und 200° durchgeführt, im allgemeinen beim Siedepunkt des Reaktionsgemisches.

Ausgangsprodukte der Formel IIa sind bekannt und oder nach bekannten Methoden herstellbar, z.B. gemäss der Europäischen Offenlegungsschrift EP-A 41 623 indem man unter basischen Bedingungen ein N-(α-Isopropyl-α-methylacetamido)-2,3-pyridincarbonsäureimid oder -phthalimid gemäss folgenden dem Schema umsetzt.

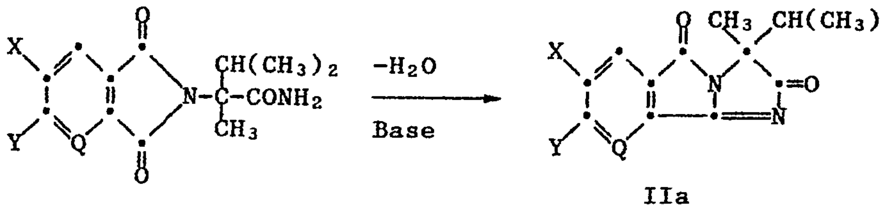


IIa

Das N-(α-Isopropyl-α-methyl-acetamido)-phthalimid kann durch Kondensation von Phthalsäureanhydrid mit 2-Amino-(α-isopropyl-α-methyl)-acetonitril erhalten werden. Anschliessend muss das Nitril durch Erwärmen in wässriger Säure zum Säureamid umgewandelt werden.

Ein Pyridin-2,3-dicarbonsäure-α-isopropyl-α-methyl-acetamid der Formel IV kann gemäss einem einfachen Verfahren hergestellt werden, indem man ein ungesättigtes Hydrazon mit einem 2-Chlor- oder 2-Brom-N-(α-isopropyl-α-methyl-acetonitril) succinimid kondensiert und eine saure Hydrolyse anschliesst, gemäss dem Schema

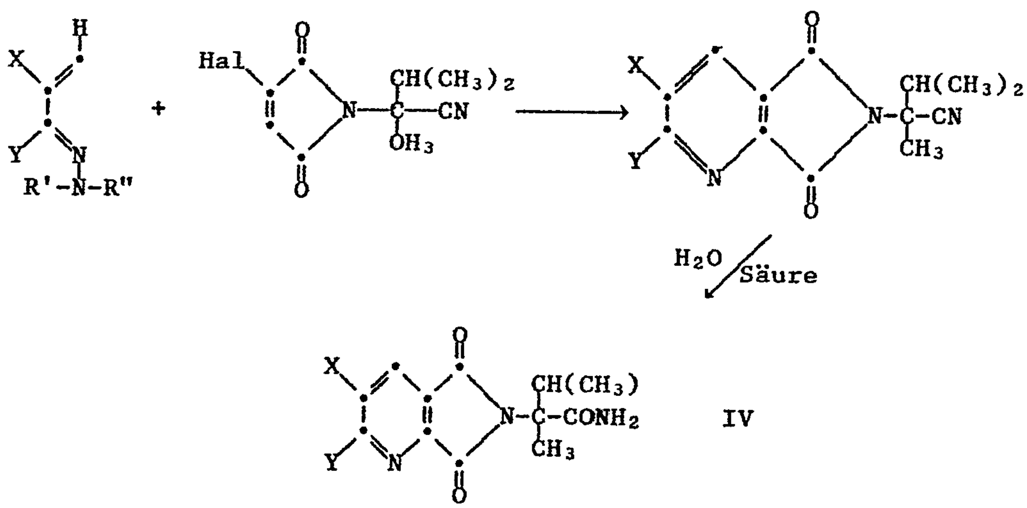


IV

In diesen Formeln bedeuten R' und R" je Wasserstoff oder $C_1$-$C_4$-Alkyl, Hal Chlor oder Brom, während X und Y die unter der Formel I gegebene Bedeutung haben.

Die Ausgangsstoffe der Formel IIb werden erhalten, indem man das obige N-(α-Isopropyl-α-methylacetamido)-2,3-pyridincarbonsäureimid oder -phthalsäureimid in Gegenwart einer Base wie z.B. Natronlauge zum 2-(4-Isopropyl-4-methyl-5-oxo-imidazolidin)-nicotinsäurederivat umwandelt.

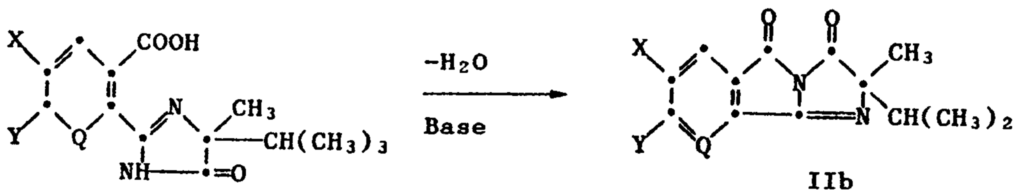


IIb

welches sich beim Behandeln mit einem wasserentziehenden Mittel in einem inerten organischen Lösungsmittel unter Verlust eines Wassermoleküls zum Ausgangsprodukt der Formel IIb (2-Isopropyl-2-methyl-3H-imidazo [1',2':1,2]pyrrolo[3,4-b]pyridin 3,5-dion) umwandelt. Solche Nicotinsäureester und ihre Herstellung sind auch in der Europäischen Offenlegungsschrift EP-A 41 623 beschrieben.

Als wasserentziehende Mittel für diese Cyclisation kommen z.B. Kochen am Wasserabschneider, eine molare Menge einer starken Säure z.B. konzentrierter Schwefelsäure oder eines Anhydrides, oder ein wasserabsorbierende Reagentien wie Cyclohexancarbodiimid, Thionylchlorid oder Phosgen in Gegenwart von etwas Dimethylformamid in Frage.

Die Reaktionen werden soweit sie sich nicht schon bei Raumtemperatur durchführen lassen bei Temperaturen zwischen 0°C und 200°C durchgeführt, d.h. man erhitzt - falls nötig - bis zum Siedepunkt des Reaktionsgemisches und kühlt wenn nötig mit Eis/Wasser oder Eis/Solebad ab.

Als Basen kommen für diese Reaktionen vor allem anorganische Basen wie Natriumhydroxid, Natriumcarbonat, Natriumhydrid, Calciumhydroxid, Calciumcarbonat, Kaliumhydroxid, Kaliumcarbonat, sowie tertiäre inorganische Basen wie Triäthylamin in Frage.

Geeignete Lösungsmittel sind z.B. polare, aprotische Lösungsmittel, die alleine oder in Mischungen aus mindestens zwei Lösungsmitteln verwendet werden können.

Unter den neuen Estern der Formel I haben sich die Nicotinsäureester als sehr aktiv erwiesen, in denen sowohl Q und $Q_1$ Stickstoff, $R_1$, $R_2$ und Y je Wasserstoff oder $C_1$-$C_4$-Alkyl bedeuten und welche der Formel Ia entsprechen

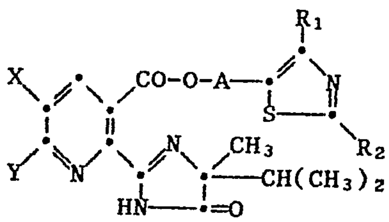

(Ia)

Speziell die Verbindungen:

2-(5-Isopropyl-5-methyl-4-oxo-4,5-dihydro-imidazolin-2-yl)-nicotinsäure-(4-methyl-thiazol-5-yl-äthyl)ester,
5-Methyl-2-(5-isopropyl-5-methyl-4-oxo-4,5-dihydro-imidazolin-2-yl)-nicotinsäure-(4-methyl-thiazol-5-yl-äthyl)ester,
5-n-Propyl-2-(5-isopropyl-5-methyl-4-oxo-4,5-dihydro-imidazolin-2-yl)-nicotinsäure-(4-methyl-thiazol-5-yl-äthyl)ester,
5-Isopropyl-2-(5-isopropyl-5-methyl-4-oxo-4,5-dihydro-imidazolin-2-yl)-nicotinsäure-(4-methyl-thiazol-5-yl-äthyl)ester.

Ebenso wirksam waren die Nicotinsäure-thienylalkylester in welchen Q Stickstoff, $Q_1$ die Methingruppe $R_1$, $R_2$, X und Y je Wasserstoff oder $C_1$-$C_4$-Alkyl bedeuten und die Formel Ib entsprechen.

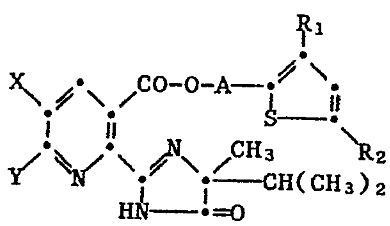

(Ib)

Speziell:
2-(5-Isopropyl-5-methyl-4-oxo-4,5-dihydro-imidazolin-2-yl)-nicotinsäure-thiophen-2-yl-äthylester,
5-Methyl-2-(5-isopropyl-5-methyl-4-oxo-4,5-dihydro-imidazolin-2-yl)-nicotinsäure-(4-methyl-thiophen-2-yl-äthyl)ester,

5-Aethyl-2-(5-isopropyl-5-methyl-4-oxo-4,5-dihydro-imidazolin-2-yl)-nicotinsäure-(4-methyl-thiophen-2-yläthyl)ester,
5-Methyl-2-(5-isopropyl-5-methyl-4-oxo-4,5-dihydro-imidazolin-2-yl)-nicotinsäure-(4-methyl-thiophen-2-ylmethyl)ester,
5-Aethyl-2-(5-isopropyl-5-methyl-4-oxo-4,5-dihydro-imidazolin-2-yl)-nicotinsäure-(4-methyl-thiophen-2-ylmethyl)ester,

Weiterhin wirksam waren die Benzoethiadiazolylalkylester, Q Methin, $Q_1$ Stickstoff und $R_1$, $R_2$, X und Y je Wasserstoff oder $C_1$-$C_4$-Alkyl oder X und Y zusammen die Butadienbrücke bedeuten und welche der Formel Ic entsprechen.

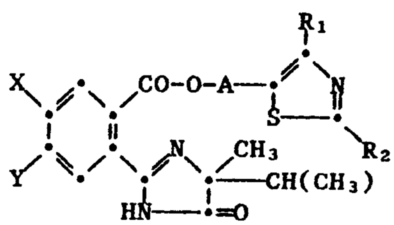

(Ic)

Als besonders wirksam hat sich der 2-(5-Isopropyl-5-methyl-4-oxo-4,5-dihydro-imidazolin-2-yl)-benzoesäure-(4-methyl-thiadiazol-5-yläthyl)ester erwiesen.

Gute Wirkung zeigen die Benzoe- oder Naphthoesäurethienylalkylester, worin Q und $Q_1$ Methin, $R_1$, $R_2$, X und Y je Wasserstoff oder $C_1$-$C_4$-Alkyl oder X und Y zusammen die Butadienbrücke bedeuten und die der Formel Ie entsprechen

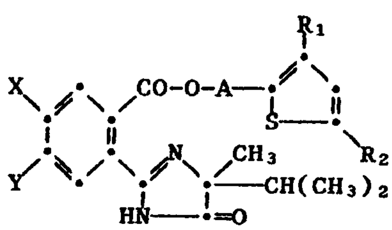

(Ie)

ferner die 3-Chinolinsäure-thiazolylalkyl und -thienylalkylester, worin Q Stickstoff, $Q_1$ Stickstoff oder Methin, $R_1$ und $R_2$ je Wasserstoff oder $C_1$-$C_4$-Alkyl und X und Y zusammen die Butadienbrücke bedeuten, entsprechen der Formel If

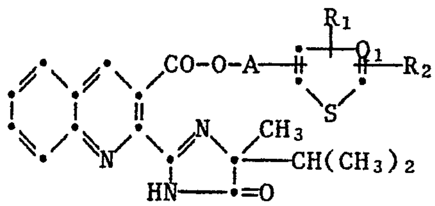

(If)

Die Erfindung betrifft alle diastereomeren und enantiomeren Isomeren der Verbindungen der Formel I.

Die Wirkstoffe der Formel I werden in der Regel mit Aufwandmengen von 0,05 bis 4 kg/ha insbesondere 0,1 bis 1 kg/ha erfolgreich eingesetzt.

Bei geringeren Aufwandmengen zeichnen sich die Verbindungen der Formel I durch gute selektiv-wuchshemmende und selektiv-herbizide Eigenschaften aus, die sie ausgezeichnet zum Einsatz in Kulturen von Nutzpflanzen, insbesondere in Getreide, Baumwolle, Soja, Mais und Reis, befähigen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d.h. sie werden von der Pflanze aufgenommen und an andere Stellen transportiert, wo sie dann zur Wirkung kommen. So gelingt es beispielsweise, durch Oberflächenbehandlung perennierende Unkräuter bis in die Wurzeln zu schädigen. Die neuen Verbindungen der Formel I wirken bereits bei - im Vergleich zu anderen Herbiziden und Wuchsregulatoren - sehr geringen Aufwandmengen.

Die Verbindungen der Formel I haben ausserdem starke pflanzenwuchshemmende Eigenschaften. Es werden sowohl Monokotyledonen als auch Dikotyledonen in ihrem Wachstum beeinträchtigt.

So können z.B. die in der Landwirtschaft in tropischen Gegenden häufig als "cover crops" (Bodenbedecker) angepflanzten Leguminosen durch die Verbindungen der Formel I in ihrem Wachstum selektiv

gehemmt werden, so dass zwar die Bodenerosion zwischen den Kulturpflanzen verhindert wird, die "cover crops" jedoch nicht zur Konkurrenz für die Kultur werden können.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten- und Fruchtbildung zugute kommen, während das vegetative Wachstum eingeschränkt wird.

Bei grösseren Aufwandmengen werden alle getesteten Pflanzen in ihrer Entwicklung so geschädigt, dass sie absterben.

Die Erfindung betrifft auch herbizide und pflanzenwachstumsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dicotylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl-oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier-und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäuremethyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 10 Glykoläthergruppen und 8 bis 20 Kohlensotffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffdatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthanol Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual"
MC Publishing Corp., Ridgewood, New Jersey, 1979.
Dr. Helmut Stache "Tensid Taschenbuch"
Carl Hanser Verlag, München/Wien 1981.

Die pestiziden Zubereitungen enthalten in der Regel 0,1 bis 95 %, insbesondere 0,1 bis 80 %, Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent)

Emulgierbare Konzentrate:

| | | |
|---|---|---|
| Wirkstoff der Formel I: | 1 bis 20 % | bevorzugt 5 bis 10 % |
| oberflächenaktives Mittel: | 5 bis 30 %, | vorzugsweise 10 bis 20 % |
| flüssiges Trägermittel: | 50 bis 94 %, | vorzugsweise 70 bis 85 %. |

Stäube:

| | | |
|---|---|---|
| Wirkstoff der Formel I: | 0,1 bis 10 %, | |
| vorzugsweise | 0,1 bis 1 % | |
| festes Trägermittel: | 99,9 bis 90 %, | vorzugsweise 99,9 bis 99 %. |

Suspensions-Konzentrate:

| | | |
|---|---|---|
| Wirkstoff der Formel I: | 5 bis 75 %, | vorzugsweise 10 bis 50 % |
| Wasser: | 94 bis 25 %, | vorzugsweise 90 bis 30 % |
| oberflächenaktives Mittel: | 1 bis 40 %, | vorzugsweise 2 bis 30 %. |

Benetzbare Pulver:

| | | |
|---|---|---|
| Wirkstoff der Formel I: | 0,5 bis 90 %, | vorzugsweise 1 bis 80 % |
| oberflächenaktives Mittel: | 0,5 bis 20 %, | vorzugsweise 1 bis 15 % |
| festes Trägermittel: | 5 bis 95 %, | vorzugsweise 15 bis 90 %. |

Granulate:

| | | |
|---|---|---|
| Wirkstoff der Formel I: | 0,5 bis 30 %, | vorzugsweise 3 bis 15 % |
| festes Trägermittel: | 99,5 bis 70 %, | vorzugsweise 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,005 bis 5 kg As/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiele veranschaulichen die Herstellung einiger Verbindungen der Formel I. Weitere in entsprechender Weise hergestellte Wirkstoffe sind in den anschliessenden Tabellen aufgeführt. Temperaturangaben beziehen sich auf Celsius-Grade, Drucke sind in Millibar angegeben.

Beispiel 1: Herstellung von 2-(5-Isopropyl-5-methyl-4-oxo-4,5-dihydro-imidazolin-2-yl)-nicotinsäure-(4-methyl-thiazol-5-yl)-äthyl)-ester

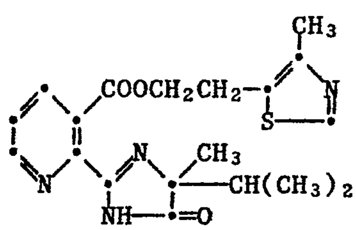

In einem Sulfierkolben wird das Gemisch von 9 g 3,5-Dioxo-2-isopropyl-2-methyl-2,3-dihydroimidazo[1',2':1,2]5-H-pyrrolo[3,4-b]-pyridin in 50 ml Tetrahydrofuran, 5,5 g 5-(2-Hydroxyäthyl)-4-methylthiazol und 2 Tropfen 1,8-Diazabicyclo[5.4.0]undec-7-en 3 Stunden am Rückfluss gekocht, dann dampft man das Lösungsmittel am Vakuum ein und verreibt den zunächst öligen Rückstand mit 50 ml Aether. Es kristallisieren 7,7 g des Produkts. Eine weitere Portion kann durch

Einengen der Mutterlauge und Kristallisation aus Aceton/Aether erhalten werden. Die Kristalle schmelzen bei 154-157°. Ausbeute 8.5 g.

Das als Ausgangsmaterial benötigte 3,5-Dioxo-2-isopropyl-2-methyl-2,3-dihydro-imidazo[1',2':1,2]5-H-pyrrolo[3,4-b]pyridin wird wie folgt hergestellt:

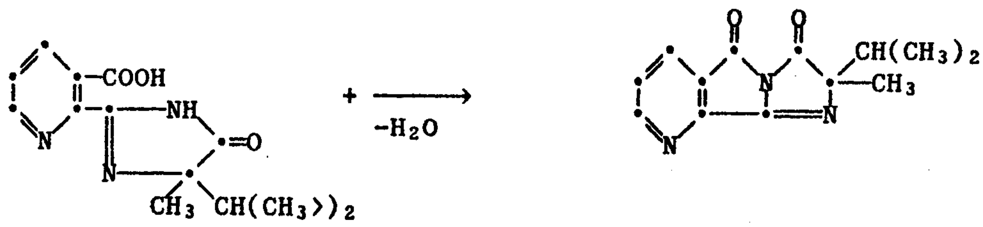


Zu einer Lösung von 11,1 g Dicyclohexylcarbodiimid in 100 ml Methylenchlorid gibt man unter Rühren 15,6 g (0.05 Mol) 2-(5-Isopropyl-5-methyl)-2-oxo-2-imidazolin-2-yl-nicotinsäure und rührt zwei Stunden bei Raumtemperatur weiter. Dann nutscht man den entstandenen Dicyclohexylharnstoff ab und wäscht den Rückstand mit wenig Methylenchlorid nach. Das Filtrat wird eingedampft und der Rückstand aus Essigsäureäthylester/Hexan umkristallisiert. Man erhält so das Titelprodukt in 91%iger Ausbeute (12.1 g) als Kristalle, die bei 132-133° schmelzen.

In analoger Weise dazu werden die folgenden Ausgangsprodukte erhalten.

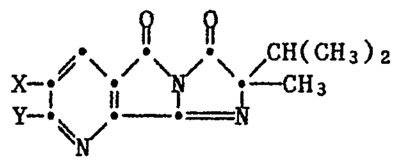

| X | X | phys. Daten |
|---|---|---|
| $nC_3H_7$ | H | Smp. 132-133° |
| $CH(CH_3)_2$ | H | Smp. 145-146° |
| $C_2H_5$ | H | Smp. 136-138° |
| $n-C_4H_9$ | H | Smp. 116-119° |
| $CH_3$ | H | Smp. 129-131° |

Beispiel 2: Herstellung von 2-(5-Isopropyl-5-methyl-4-oxo-4,5-dihydro-imidazolin-2-yl)-nicotinsäure-thienyl-2-yl-äthylester

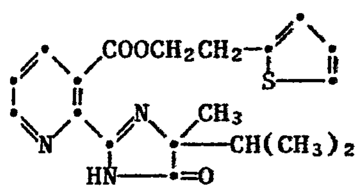

Eine Lösung von 10 g 2-(2-Thienyl)-äthanol wird in 100 ml trockenem Toluol unter Stickstoffatmosphäre langsam mit einem Gramm Natriumhydrid (80 % in Weissöl) versetzt. Nach 15 Minuten werden 10 g 3-Isopropyl-3-methyl-3H-imidazo[1',2':1.2]pyrrolo-[3,4-b]pyridin-2,5-dion zugegeben und für eine Stunde am Rückfluss erhitzt. Zur Aufarbeitung dampft man die Reaktionsmischung am Vakuum ein und chromatographiert den Rückstand an einer Kieselgel-Säule mit Tetrahydrofuran/Petroläther 1:2 als Laufmittel. Die ersten Fraktionen enthalten überschüssiges Thienyläthanol, aus den darauffolgenden wird das Produkt durch Eindampfen und Trocknen am Vakuum als helles Oel isoliert, welches beim Anreiben kristallisiert. Man erhält so 9 g Titelprodukt, welches bei 84-87° schmilzt.

Das als Ausgangsmaterial benötigte 3-Isopropyl-3-methyl-3H-imidazo-[1',2':1.2]pyrrolo-[3,4-b]pyridin-2,5-dion] wird wie folgt hergestellt:

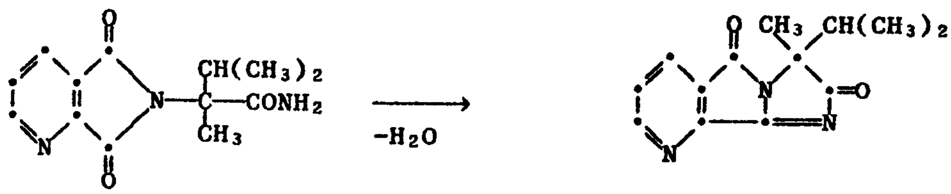


Eine Lösung von 5,7-Dihydro-α-isopropyl-α-methyl-5,7-dioxo-6H-pyrrolo-[3,4-b]pyridin-6-acetamid (Smp. 82°C) in 100 ml Toluol wird unter Zugabe von 0,5 g gepulvertem Natriumhydroxid während 2 Stunden am Wasserabschneider unter Rückfluss erhitzt. Nach dem Erkalten filtriert man die Reaktionslösung durch Kieselgel, wäscht mit Essigsäureäthylester nach und dampft das Filtrat ein. Der Rückstand wird aus Essigsäureäthylester/Petroläther auskristallisiert. Man erhält so 13,1 g der obigen Verbindung, welche bei 116°C schmilzt.

In analoger Weise dazu werden die folgenden Ausgangsverbindungen erhalten.

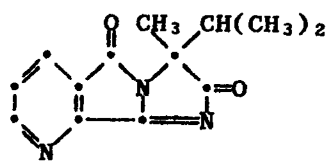

| X | Y | phys. Daten |
|---|---|---|
| $nC_3H_7$ | H | Smp. 116° |
| $C_2H_5$ | H | Smp. 119-120° |
| $n-C_4H_9$ | H | Smp. 86-87° |
| $CH(CH_3)_2$ | H | |
| $CH_3$ | H | Smp. 102-106° |
| $-(CH=CH)_2-$ | H | |
| H | $CH_3$ | |
| $CH_3$ | $CH_3$ | |
| $CH(CH_3)C_2H_5$ | H | |

In analoger Weise zu den Beispielen 1 und 2 und in sinngemässer Anwendung der entsprechenden Ausgangsprodukte werden die in den folgenden Tabellen aufgeführten Verbindungen erhalten:

Tabelle 1:

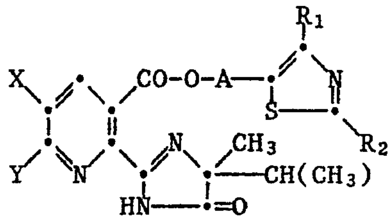

(Ia)

| Nr. | -A- | X | Y | $R_1$ | $R_2$ | phys. Daten |
|---|---|---|---|---|---|---|
| 1.01 | $-C_2H_4-$ | H | H | $CH_3$ | H | Smp. 154-157° Beispiel 1 |
| 1.02 | $-C_2H_4-$ | $CH_3$ | H | $CH_3$ | H | Smp. 103-105° |
| 1.03 | $-C_2H_4-$ | $C_2H_5$ | H | $CH_3$ | H | Smp. 97-99° |
| 1.04 | $-C_2H_4-$ | $C_3H_7$-n | H | $CH_3$ | H | Smp. 107-110° |
| 1.05 | $-C_2H_4-$ | $-(CH=CH)_2-$ | H | $CH_3$ | H | Smp. 148-150° |
| 1.06 | $-C_2H_4-$ | H | H | $CH_3$ | $CH_3$ | |
| 1.07 | $-C_2H_4-$ | H | H | $C_2H_5$ | H | |
| 1.08 | $-C_2H_4-$ | $CH_3$ | H | H | H | |
| 1.09 | $-C_2H_4-$ | $C_2H_5$ | H | H | H | |
| 1.10 | $-C_2H_4-$ | $CH_3$ | H | H | $CH_3$ | |
| 1.11 | $-C_2H_4-$ | $C_2H_5$ | H | H | $CH_3$ | |
| 1.12 | $-C_2H_4-$ | $-(CH=CH)_2-$ | | $CH_3$ | $CH_3$ | |
| 1.13 | $-C_2H_4-$ | H | $CH_3$ | $CH_3$ | H | |
| 1.14 | $-C_2H_4-$ | $C_3H_7$-i | H | $CH_3$ | H | |
| 1.15 | $-C_2H_4-$ | $C_4H_9$-n | H | $CH_3$ | H | |
| 1.16 | $-C_2H_4-$ | $CH_3$ | H | $CH_3$ | $CH_3$ | |
| 1.17 | $-C_2H_4-$ | $-(CH=CH)_2-$ | | $CH_3$ | $C_2H_5$ | |
| 1.18 | $-C_2H_4-$ | H | $CH_3$ | H | H | |
| 1.19 | $-C_2H_4-$ | H | $C_2H_5$ | H | H | |
| 1.20 | $-C_2H_4-$ | H | H | H | H | |
| 1.21 | $-C_2H_4-$ | H | $CH_3$ | $C_2H_5$ | H | |
| 1.22 | $-C_2H_4-$ | H | H | $C_2H_5$ | $CH_3$ | |
| 1.23 | $-CH_2-$ | H | H | $CH_3$ | H | |
| 1.24 | $-CH_2-$ | $CH_3$ | H | $CH_3$ | H | |
| 1.25 | $-CH_2-$ | $C_2H_5$ | H | $CH_3$ | H | |

<u>Tabelle 1</u> (Fortsetzung):

| Nr. | A | X | Y | $R_1$ | $R_2$ | phys. Daten |
|---|---|---|---|---|---|---|
| 1.26 | $-CH_2-$ | $C_3H_7$-n | H | $CH_3$ | H | |
| 1.27 | $-CH_2-$ | $-(CH=CH)_2-$ | | $CH_3$ | H | |
| 1.28 | $-CH_2-$ | H | H | $CH_3$ | $CH_3$ | |
| 1.29 | $-CH_2-$ | H | H | $C_2H_5$ | H | |
| 1.30 | $-CH_2-$ | $CH_3$ | H | H | H | |
| 1.31 | $-CH_2-$ | $C_2H_5$ | H | H | H | |
| 1.32 | $-CH_2-$ | $-(CH=CH)_2-$ | | H | $CH_3$ | |
| 1.33 | $-CH_2-$ | H | $CH_3$ | H | $CH_3$ | |
| 1.34 | $-CH_2-$ | $C_3H_7$-i | H | $CH_3$ | $CH_3$ | |
| 1.35 | $-CH_2-$ | $C_4H_9$-n | H | $CH_3$ | H | |
| 1.36 | $-CH_2-$ | $CH_3$ | H | $CH_3$ | H | |
| 1.37 | $-CH_2-$ | $-(CH=CH)_2-$ | | H | H | |
| 1.38 | $-CH_2-$ | H | $CH_3$ | $CH_3$ | $CH_3$ | |
| 1.39 | $-CH_2-$ | H | $C_2H_5$ | $CH_3$ | $C_2H_5$ | |
| 1.40 | $-CH_2-$ | H | $CH_3$ | H | H | |
| 1.41 | $-CH_2-$ | H | $C_2H_5$ | H | H | |
| 1.42 | $-CH_2-$ | H | H | H | H | |
| 1.43 | $-CH_2-$ | H | $CH_3$ | $C_2H_5$ | H | |
| 1.44 | $-CH_2-$ | H | H | $C_2H_5$ | $CH_3$ | |
| 1.45 | $-CH_2-$ | Br | H | H | H | |
| 1.46 | $-CH_2-$ | H | $C_3H_7$i | H | H | |
| 1.47 | $-CH_2-$ | Cl | H | H | H | |
| 1.48 | $-CH_2-$ | -CH=CCl-CH=CH- | | H | H | |
| 1.49 | $-CH_2-$ | H | $CH(CH_3)_2$ | H | H | |
| 1.50 | $-C_2H_4-$ | H | H | H | H | |
| 1.51 | $-C_2H_4-$ | $-CH=CH)_2-$ | | H | H | |
| 1.52 | $-C_2H_4-$ | Cl | H | H | H | |
| 1.53 | $-C_2H_4-$ | -CH=CCl-CH=CH- | | H | H | |
| 1.54 | $-C_2H_4-$ | Br | H | H | H | |

Tabelle 2:

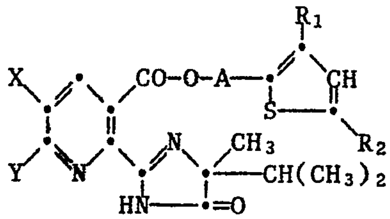

(Ib)

| Nr. | -A- | X | Y | $R_1$ | $R_2$ | phys. Daten |
|---|---|---|---|---|---|---|
| 2.01 | $-C_2H_4-$ | H | H | H | H | Smp. 84-87° Beispiel 2 |
| 2.02 | $-C_2H_4-$ | $CH_3$ | H | H | H | Smp. 110-112° |
| 2.03 | $-C_2H_4-$ | $C_2H_5$ | H | H | H | Smp. 75-78° |
| 2.04 | $-C_2H_4-$ | $C_3H_7$-n | H | $CH_3$ | H | |
| 2.05 | $-C_2H_4-$ | $-(CH{=}CH)_2-$ | | $CH_3$ | H | |
| 2.06 | $-C_2H_4-$ | H | H | $CH_3$ | $CH_3$ | |
| 2.07 | $-C_2H_4-$ | H | H | $C_2H_5$ | H | |
| 2.08 | $-C_2H_4-$ | $-(CH{=}CH)_2-$ | | H | H | |
| 2.09 | $-C_2H_4-$ | $C_3H_7$i | H | H | H | |
| 2.10 | $-C_2H_4-$ | $CH_3$ | H | H | $CH_3$ | |
| 2.11 | $-C_2H_4-$ | $C_2H_5$ | H | H | $CH_3$ | |
| 2.12 | $-C_2H_4-$ | $-(CH{=}CH)_2-$ | | $CH_3$ | $CH_3$ | |
| 2.13 | $-C_2H_4-$ | H | $CH_3$ | $CH_3$ | H | |
| 2.14 | $-C_2H_4-$ | $C_3H_7$-i | H | $CH_3$ | H | |
| 2.15 | $-C_2H_4-$ | $C_4H_9$-n | H | $CH_3$ | H | |
| 2.16 | $-C_2H_4-$ | $CH_3$ | H | $CH_3$ | $CH_3$ | |
| 2.17 | $-C_2H_4-$ | $-(CH{=}CH)_2-$ | | $CH_3$ | $C_2H_5$ | |
| 2.18 | $-C_2H_4-$ | H | $CH_3$ | H | H | Smp. 115-116° |
| 2.19 | $-C_2H_4-$ | H | $C_2H_5$ | H | H | |
| 2.20 | $-C_2H_4-$ | $-CH{=}CCl-CH{=}CH_2-$ | | H | H | |
| 2.21 | $-C_2H_4-$ | H | $CH_3$ | $C_2H_5$ | H | |
| 2.22 | $-C_2H_4-$ | H | H | $C_2H_5$ | $CH_3$ | |
| 2.23 | $-CH_2-$ | H | H | H | H | Smp. 126-127° |
| 2.24 | $-CH_2-$ | $CH_3$ | H | H | H | Smp. 121-123° |
| 2.25 | $-CH_2-$ | $C_2H_5$ | H | H | H | Smp. 87-88,5° |
| 2.26 | $-CH_2-$ | $C_3H_7$-n | H | $CH_3$ | H | |

Tabelle 2 (Fortsetzung):

| Nr. | -A- | X | Y | $R_1$ | $R_2$ | phys. Daten |
|---|---|---|---|---|---|---|
| 2.27 | $-CH_2-$ | $-(CH=CH)_2-$ | | $CH_3$ | H | |
| 2.28 | $-CH_2-$ | H | H | $CH_3$ | $CH_3$ | |
| 2.29 | $-CH_2-$ | H | H | $C_2H_5$ | H | |
| 2.30 | $-CH_2-$ | Cl | H | H | H | |
| 2.31 | $-CH_2-$ | $-(CH=CH)_2-$ | | H | $CH_3$ | |
| 2.32 | $-CH_2-$ | $CH_3$ | H | H | $CH_3$ | |
| 2.33 | $-CH_2-$ | $C_2H_5$ | H | H | $CH_3$ | |
| 2.34 | $-CH_2-$ | $-(CH=CH)_2-$ | | $CH_3$ | $CH_3$ | |
| 2.35 | $-CH_2-$ | H | $CH_3$ | $CH_3$ | H | |
| 2.36 | $-CH_2-$ | $C_3H_7$-i | H | $CH_3$ | H | |
| 2.37 | $-CH_2-$ | $C_4H_9$-n | H | $CH_3$ | H | |
| 2.38 | $-CH_2-$ | $CH_3$ | H | $CH_3$ | $CH_3$ | |
| 2.39 | $-CH_2-$ | $-(CH=CH)_2-$ | | $CH_3$ | $C_2H_5$ | |
| 2.40 | $-CH_2-$ | H | $CH_3$ | H | H | |
| 2.41 | $-CH_2-$ | H | $C_2H_5$ | H | H | |
| 2.42 | $-CH_2-$ | H | H | H | H | |
| 2.43 | $-CH_2-$ | H | $CH_3$ | $C_2H_5$ | H | |
| 2.44 | $-CH_2-$ | H | H | $C_2H_5$ | $CH_3$ | |
| 2.45 | $-CH_2-$ | $-(CH=CH_2)_2-$ | | H | $CH_3$ | |
| 2.46 | $-CH_2-$ | Br | H | H | $CH_3$ | |
| 2.47 | $-CH_2-$ | Cl | H | H | $CH_3$ | |
| 2.48 | $-CH_2-$ | $-CH=CCl-CH=CH-$ | | H | $CH_3$ | |
| 2.49 | $-CH_2-$ | H | $C_3H_7$i | H | $CH_3$ | |
| 2.50 | $-CH_2-$ | $-(CH=CH)_2$ | | H | H | Smp. 136-137° |
| 2.51 | $-CH_2-$ | Cl | H | H | H | |
| 2.52 | $-CH_2-$ | $-CH=CCl-CH=CH_2-$ | | H | H | |
| 2.53 | $-CH_2-$ | Br | H | H | H | |
| 2.54 | $-CH_2-$ | H | $CH(CH_3)_2$ | H | H | |
| 2.55 | $-CH(C_3H_7n)-$ | H | H | H | H | |
| 2.56 | $-CH(C_3H_7n)-$ | $CH_3$ | H | H | H | |
| 2.57 | $-CH(C_3H_7n)-$ | $C_2H_5$ | H | H | H | |
| 2.58 | $-CH(C_3H_7n)-$ | $-(CH=CH)_2-$ | | H | H | |

Tabelle 2 (Fortsetzung)

| Nr. | -A- | X | Y | $R_1$ | $R_2$ | phys.Daten |
|---|---|---|---|---|---|---|
| 2.59 | $-CH(C_3H_7n)-$ | Cl | H | H | H | |
| 2.60 | $-CH(C_3H_7n)-$ | -CH=CCl-CH=CH- | | H | H | |
| 2.61 | $-CH(C_3H_7n)-$ | Br | H | H | H | |
| 2.62 | $-CH(C_3H_7n)-$ | H | $CH(CH_3)_2$ | H | H | |
| 2.63 | $-CH(CH_3)-$ | H | H | H | H | |
| 2.64 | $-CH(CH_3)-$ | $CH_3$ | H | H | H | |
| 2.65 | $-CH(CH_3)-$ | $C_2H_5$ | H | H | H | |
| 2.66 | $-CH(CH_3)_2-$ | $-(CH=CH)_2-$ | | H | H | |
| 2.67 | $-CH(CH_3)_2-$ | Cl | H | H | H | |
| 2.68 | $-CH(CH_3)_2-$ | -CH=CCl-CH=CH- | | H | H | |
| 2.69 | $-CH(CH_3)_2-$ | Br | H | H | H | |
| 2.70 | $-CH(CH_3)_2-$ | H | $CH(CH_3)_2$ | H | H | |
| 2.71 | $-CH_2-$ | H | H | H | $CH_3$ | |
| 2.72 | $-(CH_2)_3-$ | H | H | H | H | |
| 2.73 | $-CH_2CH_2-$ | Br | H | H | H | |
| 2.74 | $-CH_2CH_2$ | Cl | H | H | H | |
| 2.75 | $-C_2H_4-$ | Br | H | H | H | |
| 2.76 | $-C_2H_4-$ | H | $CH(CH_3)_2$ | H | H | |
| 2.77 | $-(CH_2)_3-$ | H | H | H | H | |
| 2.78 | $-(CH_2)_3-$ | $CH_3$ | H | H | H | |
| 2.79 | $-(CH_2)_3-$ | $C_2H_5$ | H | H | H | |
| 2.80 | $-(CH_2)_3-$ | $-(CH=CH)_2-$ | | H | H | |
| 2.81 | $-(CH_2)_3-$ | Cl | H | H | H | |
| 2.82 | $-(CH_2)_3-$ | -CH=CCl-CH=CH- | | H | H | |
| 2.83 | $-(CH_2)_3-$ | Br | H | H | H | |
| 2.84 | $-(CH_2)_3-$ | H | $CH(CH_3)_2$ | H | H | |
| 2.85 | $-CH_2-$ | $CH_3$ | H | H | $CH_3$ | |
| 2.86 | $-CH_2-$ | $C_2H_5$ | H | H | $CH_3$ | |
| 2.87 | $-CH_2-$ | $-(CH=CH)_2-$ | | H | $CH_3$ | |
| 2.88 | $-CH_2-$ | -CH=CCl-CH=CH- | | H | $CH_3$ | |
| 2.89 | $-CH_2-$ | Br | H | H | $CH_3$ | |
| 2.90 | $-CH_2-$ | H | $CH(CH_3)_2$ | H | $CH_3$ | |

Tabelle 3:

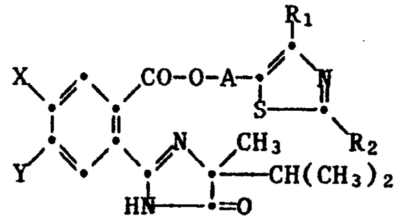

(Ic)

| Nr. | -A- | X | Y | $R_1$ | $R_2$ | phys. Daten |
|---|---|---|---|---|---|---|
| 3.01 | $-C_2H_4-$ | H | H | $CH_3$ | H | Oel $n_D^{25}$ = 1.5624 |
| 3.02 | $-C_2H_4-$ | $CH_3$ | H | $CH_3$ | H | |
| 3.03 | $-C_2H_4-$ | $C_2H_5$ | H | $CH_3$ | H | |
| 3.04 | $-C_2H_4-$ | $C_3H_7$-n | H | $CH_3$ | H | |
| 3.05 | $-C_2H_4-$ | $-(CH{=}CH)_2-$ | | $CH_3$ | H | |
| 3.06 | $-C_2H_4-$ | H | H | $CH_3$ | $CH_3$ | |
| 3.07 | $-C_2H_4-$ | H | H | $C_2H_5$ | H | |
| 3.08 | $-C_2H_4-$ | $CH_3$ | H | H | H | |
| 3.09 | $-C_2H_4-$ | $C_2H_5$ | H | H | H | |
| 3.10 | $-C_2H_4-$ | $CH_3$ | H | H | $CH_3$ | |
| 3.11 | $-C_2H_4-$ | $C_2H_5$ | H | H | $CH_3$ | |
| 3.12 | $-C_2H_4-$ | $-(CH{=}CH)_2-$ | | $CH_3$ | $CH_3$ | |
| 3.13 | $-C_2H_4-$ | H | $CH_3$ | $CH_3$ | H | |
| 3.14 | $-C_2H_4-$ | $C_3H_7$-i | H | $CH_3$ | H | |
| 3.15 | $-C_2H_4-$ | $C_4H_9$-n | H | $CH_3$ | H | |
| 3.16 | $-C_2H_4-$ | $CH_3$ | H | $CH_3$ | $CH_3$ | |
| 3.17 | $-C_2H_4-$ | $-(CH{=}CH)_2-$ | | $CH_3$ | $C_2H_5$ | |
| 3.18 | $-C_2H_4-$ | H | $CH_3$ | H | H | |
| 3.19 | $-C_2H_4-$ | H | $C_2H_5$ | H | H | |
| 3.20 | $-C_2H_4-$ | H | H | H | H | |
| 3.21 | $-C_2H_4-$ | H | $CH_3$ | $C_2H_5$ | H | |
| 3.22 | $-C_2H_4-$ | H | H | $C_2H_5$ | $CH_3$ | |
| 3.23 | $-CH_2-$ | H | H | $CH_3$ | H | |
| 3.24 | $-CH_2-$ | $CH_3$ | H | $CH_3$ | H | |
| 3.25 | $-CH_2-$ | $C_2H_5$ | H | $CH_3$ | H | |
| 3.26 | $-CH_2-$ | $C_3H_7$-n | H | $CH_3$ | H | |
| 3.27 | $-CH_2-$ | $-CH{=}CCl-CH{=}CH-$ | | $CH_3$ | H | |
| 3.28 | $-CH_2-$ | H | H | $CH_3$ | $CH_3$ | |

<u>Tabelle 3</u> (Fortsetzung):

| Nr. | -A- | X | Y | $R_1$ | $R_2$ | phys. Daten |
|---|---|---|---|---|---|---|
| 3.29 | $-CH_2-$ | H | H | $C_2H_5$ | H | |
| 3.30 | $-CH_2-$ | $CH_3$ | H | H | H | |
| 3.31 | $-CH_2-$ | $C_2H_5$ | H | H | H | |
| 3.32 | $-CH_2-$ | $CH_3$ | H | H | $CH_3$ | |
| 3.33 | $-CH_2-$ | $C_2H_5$ | H | H | $CH_3$ | |
| 3.34 | $-CH_2-$ | $-(CH{=}CH)_2-$ | | $CH_3$ | $CH_3$ | |
| 3.35 | $-CH_2-$ | H | $CH_3$ | $CH_3$ | H | |
| 3.36 | $-CH_2-$ | $C_3H_7$-i | H | $CH_3$ | H | |
| 3.37 | $-CH_2-$ | $C_4H_9$-n | H | $CH_3$ | H | |
| 3.38 | $-CH_2-$ | $CH_3$ | H | $CH_3$ | $CH_3$ | |
| 3.39 | $-CH_2-$ | $-(CH{=}CH)_2-$ | | $CH_3$ | $C_2H_5$ | |
| 3.40 | $-CH_2-$ | H | $CH_3$ | H | H | |
| 3.41 | $-CH_2-$ | H | $C_2H_5$ | H | H | |
| 3.42 | $-CH_2-$ | H | H | H | H | |
| 3.43 | $-CH_2-$ | H | $CH_3$ | $C_2H_5$ | H | |
| 3.44 | $-CH_2-$ | H | H | $C_2H_5$ | $CH_3$ | |
| 3.45 | $-CH_2-$ | $-(CH{=}CH)_2-$ | | H | H | |
| 3.46 | $-CH_2-$ | Br | H | H | H | |
| 3.47 | $-CH_2-$ | Cl | H | H | H | |
| 3.48 | $-CH_2-$ | -CH=CH-CH=CH-<br>Cl | | H | H | |
| 3.49 | $-CH_2-$ | H | $CH(CH_3)_2$ | H | H | |
| 3.50 | $-CH_2-$ | H | H | H | $CF_3$ | |
| 3.51 | $-CH_2-$ | $CH_3$ | H | H | $CF_3$ | |
| 3.52 | $-CH_2-$ | $C_2H_5$ | H | H | $CF_3$ | |
| 3.53 | $-CH_2-$ | $-(CH{=}CH)_2-$ | | H | $CF_3$ | |
| 3.54 | $-CH_2-$ | Cl | H | H | H | |
| 3.55 | $-CH_2-$ | -CH=CCl-CH=CH- | | H | H | |
| 3.56 | $-CH_2-$ | Br | H | H | H | |
| 3.57 | $-CH_2-$ | H | $CH(CH_3)_2$ | H | H | |
| 3.58 | $-CH_2CH_2-$ | Br | H | $CH_3$ | H | |
| 3.59 | $-CH_2CH_2-$ | -CH=CCl-CH=CH- | | $CH_3$ | H | |
| 3.60 | $CH_2$ | Br | H | $CH_3$ | H | |
| 3.61 | $CH_2$ | -CH=CCl-CH=CH- | | $CH_3$ | H | |

Tabelle 4:

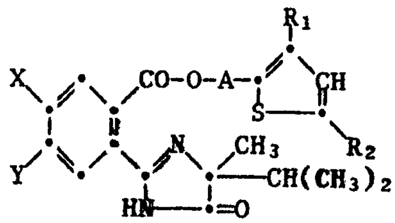

(Id)

| Nr. | -A- | X | Y | $R_1$ | $R_2$ | phys. Daten |
|---|---|---|---|---|---|---|
| 4.01 | $-C_2H_4-$ | H | H | | H | Smp. 103-105° |
| 4.02 | $-C_2H_4-$ | $CH_3$ | H | $CH_3$ | H | |
| 4.03 | $-C_2H_4-$ | $C_2H_5$ | H | $CH_3$ | H | |
| 4.04 | $-C_2H_4-$ | $C_3H_7$-n | H | $CH_3$ | H | |
| 4.05 | $-C_2H_4-$ | $-CH_2{=}CH)_2-$ | | $CH_3$ | H | |
| 4.06 | $-C_2H_4-$ | H | H | $CH_3$ | $CH_3$ | |
| 4.07 | $-C_2H_4-$ | H | H | $C_2H_5$ | H | |
| 4.08 | $-C_2H_4-$ | $CH_3$ | H | H | H | |
| 4.09 | $-C_2H_4-$ | $C_2H_5$ | H | H | H | |
| 4.10 | $-C_2H_4-$ | $CH_3$ | H | H | $CH_3$ | |
| 4.11 | $-C_2H_4-$ | $C_2H_5$ | H | H | $CH_3$ | |
| 4.12 | $-C_2H_4-$ | $-(CH{=}CH)_2-$ | | $CH_3$ | $CH_3$ | |
| 4.13 | $-C_2H_4-$ | H | $CH_3$ | $CH_3$ | H | |
| 4.14 | $-C_2H_4-$ | $C_3H_7$-i | H | $CH_3$ | H | |
| 4.15 | $-C_2H_4-$ | $C_4H_9$-n | H | $CH_3$ | H | |
| 4.16 | $-C_2H_4-$ | $CH_3$ | H | $CH_3$ | $CH_3$ | |
| 4.17 | $-C_2H_4-$ | $-(CH{=}CH)_2-$ | | $CH_3$ | $C_2H_5$ | |
| 4.18 | $-C_2H_4-$ | H | $CH_3$ | H | H | |
| 4.19 | $-C_2H_4-$ | H | $C_2H_5$ | H | H | |
| 4.20 | $-C_2H_4-$ | H | H | H | H | |
| 4.21 | $-C_2H_4-$ | H | $CH_3$ | $C_2H_5$ | H | |
| 4.22 | $-C_2H_4-$ | H | H | $C_2H_5$ | $CH_3$ | |

Tabelle 4 (Fortsetzung):

| Nr. | -A- | X | Y | $R_1$ | $R_2$ | phys. Daten |
|---|---|---|---|---|---|---|
| 4.23 | $-CH_2-$ | H | H | H | H | |
| 4.24 | $-CH_2-$ | $CH_3$ | H | H | H | |
| 4.25 | $-CH_2-$ | $C_2H_5$ | H | $CH_3$ | H | |
| 4.26 | $-CH_2-$ | $C_3H_7$-n | H | $CH_3$ | H | |
| 4.27 | $-CH_2-$ | $-CH_2=CH)_2-$ | | $CH_3$ | H | Smp. |
| 4.28 | $-CH_2-$ | H | H | $CH_3$ | $CH_3$ | |
| 4.29 | $-CH_2-$ | H | H | $C_2H_5$ | H | |
| 4.30 | $-CH_2-$ | $CH_3$ | H | H | H | |
| 4.31 | $-CH_2-$ | $C_2H_5$ | H | H | H | |
| 4.32 | $-CH_2-$ | $CH_3$ | H | H | $CH_3$ | |
| 4.33 | $-CH_2-$ | $C_2H_5$ | H | H | $CH_3$ | |
| 4.34 | $-CH_2-$ | $-(CH=CH)_2-$ | | $CH_3$ | $CH_3$ | |
| 4.35 | $-CH_2-$ | H | $CH_3$ | $CH_3$ | H | |
| 4.36 | $-CH_2-$ | $C_3H_7$-i | H | $CH_3$ | H | |
| 4.37 | $-CH_2-$ | $C_4H_9$-n | H | $CH_3$ | H | |
| 4.38 | $-CH_2-$ | $CH_3$ | H | $CH_3$ | $CH_3$ | |
| 4.39 | $-CH_2-$ | $-(CH=CH)_2-$ | | $CH_3$ | $C_2H_5$ | |
| 4.40 | $-CH_2-$ | H | $CH_3$ | H | H | |
| 4.41 | $-CH_2-$ | H | $C_2H_5$ | H | H | |
| 4.42 | $-CH_2-$ | H | H | H | H | |
| 4.43 | $-CH_2-$ | H | $CH_3$ | $C_2H_5$ | H | |
| 4.44 | $-CH_2-$ | H | H | $C_2H_5$ | $CH_3$ | |

Tabelle 5:

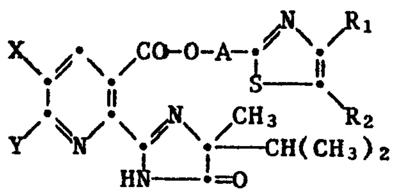

| Nr. | -A- | X | Y | $R_1$ | $R_2$ | phys.Daten |
|---|---|---|---|---|---|---|
| 5.01 | $-CH_2-$ | H | H | H | $CH_3$ | |
| 5.02 | $-CH_2-$ | $CH_3$ | H | H | $CH_3$ | |
| 5.03 | $-CH_2-$ | $C_2H_5$ | H | H | $CH_3$ | |
| 5.04 | $-CH_2-$ | $-(CH=CH)_2-$ | | H | $CH_3$ | |
| 5.05 | $-CH_2-$ | Cl | H | H | $CH_3$ | |
| 5.06 | $-CH_2-$ | -CH=CCl-CH=CH- | | H | $CH_3$ | |
| 5.07 | $-CH_2-$ | Br | H | H | $CH_2$ | |
| 5.08 | $-CH_2-$ | H | $CH(CH_3)_2$ | H | $CH_2$ | |
| 5.09 | $-CH(C_3H_7n)-$ | H | H | H | H | |
| 5.10 | $-CH(C_3H_7n)-$ | $CH_3$ | H | H | H | |
| 5.11 | $-CH_3(C_3H_7n)-$ | $C_2H_5$ | H | H | H | |
| 5.12 | $-CH(C_3H_7n)-$ | $-(CH=CH)_2-$ | | H | H | |
| 5.13 | $-CH(C_3H_7n)-$ | Cl | H | H | H | |
| 5.14 | $-CH(C_3H_7n)-$ | -CH=CCl-CH=CH- | | H | H | |
| 5.15 | $-CH(C_3H_7n)-$ | Br | H | H | H | |
| 5.16 | $-CH(C_3H_7n)-$ | H | $CH(CH_3)_2$ | H | H | |
| 5.17 | $-C_2H_4-$ | H | H | H | H | |
| 5.18 | $-C_2H_4-$ | $CH_3$ | H | H | H | |
| 5.19 | $-C_2H_4-$ | $C_2H_5$ | H | H | H | |
| 5.20 | $-C_2H_4-$ | $-(CH=CH)_2-$ | | H | H | |

Tabelle 5 (Fortsetzung)

| Nr. | -A- | X | Y | $R_1$ | $R_2$ | phys.Daten |
|---|---|---|---|---|---|---|
| 5.21 | $-C_2H_4-$ | Cl | H | H | H | |
| 5.22 | $-C_2H_4-$ | -CH=CCl-CH=CH- | | H | H | |
| 5.23 | $-C_2H_4-$ | Br | H | H | H | |
| 5.24 | $-C_2H_4-$ | H | $CH(CH_3)_2$ | H | H | |
| 5.25 | $-CH_2-$ | H | H | H | H | |
| 5.26 | $-CH_2-$ | $CH_3$ | H | H | H | |
| 5.27 | $-CH_2-$ | $C_2H_5$ | H | H | H | |
| 5.28 | $-CH_2-$ | $-(CH=CH)_2-$ | | H | H | |
| 5.29 | $-CH_2-$ | Cl | H | H | H | |
| 5.30 | $-CH_2-$ | -CH=CCl-CH=CH- | | H | H | |
| 5.31 | $-CH_2-$ | Br | H | H | H | |
| 5.32 | $-CH_2-$ | H | $CH(CH_3)_2$ | H | H | |
| 5.33 | $-CH(C_2H_5)-$ | H | H | H | H | |
| 5.34 | $-CH(C_2H_5)-$ | $CH_3$ | H | H | H | |
| 5.35 | $-CH(C_2H_5)-$ | $C_2H_5$ | H | H | H | |
| 5.36 | $-CH(C_2H_5)-$ | Cl | H | H | H | |
| 5.37 | $-CH(C_2H_5)-$ | -CH=CCl-CH=CH- | | H | H | |
| 5.38 | $-CH(C_2H_5)-$ | Br | H | H | H | |
| 5.39 | $-CH(C_2H_5)-$ | H | $CH(CH_3)_2$ | H | H | |
| 5.40 | $-CH(C_2H_5)-$ | $-(CH=CH)_2-$ | | H | H | |

Tabelle 6:

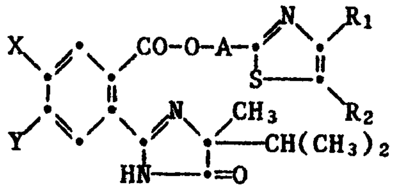

| Nr. | -A- | X | Y | $R_1$ | $R_2$ | phys.Daten |
|---|---|---|---|---|---|---|
| 6.01 | $-CH_2-$ | H | H | H | H | |
| 6.02 | $-CH_2-$ | $CH_3$ | H | H | H | |
| 6.03 | $-CH_2-$ | $C_2H_5$ | H | H | H | |
| 6.04 | $-CH_2-$ | $-(CH=CH)_2-$ | | H | H | |
| 6.05 | $-CH_2-$ | Cl | H | H | H | |
| 6.06 | $-CH_2-$ | $-CH=CCl-CH=CH-$ | | H | H | |
| 6.07 | $-CH_2-$ | Br | H | H | H | |
| 6.08 | $-CH_2-$ | H | $CH(CH_3)_2$ | H | H | |
| 6.09 | $-C_2H_4-$ | H | H | H | H | |
| 6.10 | $-C_2H_4-$ | $CH_3$ | H | H | H | |
| 6.11 | $-C_2H_4-$ | $C_2H_5$ | H | H | H | |
| 6.12 | $-C_2H_4-$ | Cl | H | H | H | |
| 6.13 | $-C_2H_4-$ | $-CH=CCl-CH=CH-$ | | H | H | |
| 6.14 | $-C_2H_4-$ | Br | H | H | H | |
| 6.15 | $-C_2H_4-$ | H | $CH(CH_3)_2$ | H | H | |
| 6.16 | $-C_2H_4-$ | $-(CH=CH)_2-$ | | H | H | |
| 6.17 | $-(CH_2)_3-$ | H | H | H | H | |
| 6.18 | $-(CH_2)_3-$ | $CH_3$ | H | H | H | |
| 6.19 | $-(CH_2)_3-$ | $C_2H_5$ | H | H | H | |
| 6.20 | $-(CH_2)_3-$ | $-(CH=CH)_2-$ | | H | H | |
| 6.21 | $-(CH_2)_3-$ | Cl | H | H | H | |
| 6.22 | $-(CH_2)_3-$ | $-CH=CCl-CH=CH-$ | | H | H | |
| 6.23 | $-(CH_2)_3-$ | Br | H | H | H | |
| 6.24 | $-(CH_2)_3-$ | H | $CH(CH_3)_2$ | H | H | |
| 6.25 | $-CH_2-$ | H | H | H | $CH_3$ | |
| 6.26 | $-CH_2-$ | $CH_3$ | H | H | $CH_3$ | |
| 6.27 | $-CH_2-$ | $C_2H_5$ | H | H | $CH_3$ | |

Tabelle 6 (Fortsetzung)

| Nr. | -A- | X | Y | $R_1$ | $R_2$ | phys.Daten |
|---|---|---|---|---|---|---|
| 6.28 | $-CH_2-$ | $-(CH{=}CH)_2-$ | | H | $CH_3$ | |
| 6.29 | $-CH_2-$ | Cl | H | H | H | |
| 6.30 | $-CH_2-$ | $CH{=}CCl-CH{=}CH-$ | | H | $CH_3$ | |
| 6.31 | $-CH_2-$ | Br | H | H | $CH_3$ | |
| 6.32 | $-CH_2-$ | H | $CH(CH_3)_2$ | H | $CH_3$ | |

Formulierungsbeispiele:

Beispiel 3: Formulierungsbeispiele für Wirkstoffe der Formel I (% = Gewichtsprozent)

a) Spritzpulver

| | a) | b) | c) |
|---|---|---|---|
| Wirkstoff gemäss Tabellen 1-6 | 20 % | 60 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyäthylenglykoläther (7-8 Mol AeO) | - | 2 % | 2 % |
| Hochdisperse Kieselsäure | 5 % | 27 % | 27 % |
| Kaolin | 67 % | - | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

b) Emulsions-Konzentrat

| | a) | b) |
|---|---|---|
| Wirkstoff gemäss Tabellen 1-6 | 10 % | 1 % |
| Octylphenolpolyäthylenglykoläther (4-5 Mol AeO) | 3 % | 3 % |
| Ca-Dodecylbenzolsulfonat | 3 % | 3 % |
| Ricinusölpolyglykoläther (36 Mol AeO) | 4 % | 4 % |
| Cyclohexanon | 30 % | 10 % |

| | | |
|---|---|---|
| Xylolgemisch | 50 % | 79 % |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff gemäss Tabellen 1-6 | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

| d) Extruder Granulat | a) | b) |
|---|---|---|
| Wirkstoff gemäss Tabellen 1-6 | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

| e) Umhüllungs-Granulat | |
|---|---|
| Wirkstoff gemäss Tabellen 1-6 | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| f) Suspensions-Konzentrat | a) | b) |
|---|---|---|
| Wirkstoff gemäss Tabellen 1-6 | 40 % | 5 % |
| Aethylenglykol | 10 % | 10 % |
| Nonylphenolpolyäthylenglykoläther (15 Mol AeO) | 6 % | 1 % |

| | | |
|---|---|---|
| Na-Ligninsulfonat | 10 % | 5 % |
| Carboxymethylcellulose | 1 % | 1 % |
| 37 %ige wässrige Formaldehyd-Lösung | 0,2 % | 0,2 % |
| Silikonöl in Form einer 75 %igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g) Salzlösung

| | |
|---|---|
| Wirkstoff gemäss Tabellen 1-7 | 5 % |
| Isopropylamin | 1 % |
| Octylphenolpolyäthylenglykoläther (78 Mol AeO) | 3 % |
| Wasser | 91 % |

Biologische Beispiele:

Beispiel 4: Pre-emergente Herbizid-Wirkung

Im Gewächshaus wird unmittelbar nach der Einsaat der Versuchspflanzen in Saatschalen die Erdoberfläche mit einer wässrigen Dispersion der Wirkstoffe, erhalten aus einem 25 %igen Emulsionskonzentrat behandelt. Es werden Konzentrationen von 4 kg Wirksubstanz/Hektar angewendet. Die Saatschalen werden im Gewächshaus bei 22-25°C und 50-70 % relativer Luftfeuchtigkeit gehalten und der Versuch nach 3 Wochen ausgewertet und die Resultate nach folgender Notenskala ausgewertet.

1 Pflanze hat nicht gekeimt oder ist eingegangen
2-3 sehr starke Schäden
4 starke Schäden
5 mittlere Schäden, die Pflanzen sind verkümmert
6 Schäden, die Pflanze kann regenerieren

7-8 leichte Schäden

9 normales Wachstum, wie unbehandelte Pflanzen

In diesem Versuch zeigten die Verbindungen der Tabellen 3-8 starke Herbizidwirkung. Einige Resultate sind in der Tabelle 7 zusammengefasst.

Tabelle 7

| Verb. Nr. | Avena | Setaria | Sinapsis | Stellaria |
|---|---|---|---|---|
| 1.01 | 2 | 1 | 2 | 2 |
| 2.01 | 2 | 2 | 1 | 2 |
| 3.01 | 2 | 1 | 2 | 2 |

Beispiel 5: Post-emergente Herbizid-Wirkung (Kontaktherbizid)

Eine Anzahl Unkräuter, sowohl monokotyle wie dikotyle, wurden nach dem Auflaufen (im 4- bis 6-Blattstadium) mit einer wässrigen Wirkstoffdispersion in einer Dosierung von 4 kg Wirksubstanz pro Hektar auf die Pflanzen gespritzt und diese bei 24°-26°C und 45-60 % relativer Luftfeuchtigkeit gehalten. 15 Tage nach der Behandlung wird der Versuch nach der obigen Notenskala ausgewertet. Auch in diesem Versuch zeigen die Verbindungen der Tabellen 1-6 starke bis sehr starke Herbizidwirkung. Einige Resultate sind in der Tabelle 8 zusammengefasst.

Tabelle 8

| Verb. No. | Avena | Setaria | Lolium | Solanum | Sinapsis | Stellaria |
|---|---|---|---|---|---|---|
| 1.01 | 1 | 3 | 1 | 1 | 2 | 2 |
| 2.01 | 1 | 2 | 2 | 1 | 2 | 2 |
| 3.02 | 1 | 2 | 2 | 1 | 1 | 2 |

Beispiel 6: Herbizidwirkung vor dem Auflaufen der Pflanzen

Kunststofftöpfe werden mit expandiertem Vermiculit (Dichte: 0,135 g/cm³, Wasserabsorptionsvermögen: 0,565 l/l) gefüllt. Nach dem Sättigen des nicht adsorptiven Vermiculits mit einer wässrigen Wirkstoffemulsion in deinonisiertem Wasser, die die Wirkstoffe in einer Konzentration von 70,8 ppm enthält, werden Samen der folgenden Pflanzen auf die Oberfläche gesät. Nasturtium offcinalis, Agrostis tenuis, Stellaria media und Digitaria sanguinalis. Die Versuchsgefässe werden anschliessend in einer Klimakammer bei 20°C, einer Beleuchtung von ca. 20 kLux und einer relativen Luftfeuchtigkeit von 70 % gehalten. Während der Keimphase von 4 bis 6 Tagen werden die Töpfe zur Erhöhung der örtlichen Luftfeuchtigkeit mit lichtdurchlässigem Material abgedeckt und mit deinoisiertem Wasser begossen. Nach dem 5. Tag wird dem Giesswasser 0,5 % eines handelsüblichen Flüssigdüngers (®Greenzit) zugesetzt. 12 Tage nach der Aussaat wird der Versuch ausgewertet und die Wirkung auf die Versuchspflanzen nach dem in Beispiel 4 gegebenen Massstab bewertet.

Die Resultate sind in der Tabelle 9 zusammengefasst:

Tabelle 9

| Verb. No. | Aufwand Menge ppm | Nasturtium officinalis | Agrostis tenuis | Stellaria media | Digitaria sang. |
|---|---|---|---|---|---|
| 1.01 | 100 | 1 | 1 | 1 | 1 |
| | 10 | 2 | 2 | 1 | 1 |
| | 1 | 3 | 2 | 1 | 3 |
| | 0.1 | 4 | 4 | 2 | 3 |
| | 0.01 | 4 | 4 | 5 | 6 |
| 2.01 | 100 | 2 | 2 | 2 | 2 |
| | 10 | 2 | 2 | 2 | 2 |
| | 1 | 2 | 2 | 2 | 2 |
| | 0.1 | 3 | 3 | 3 | 3 |
| | 0.01 | 3 | 3 | 3 | 3 |
| 3.01 | 100 | 2 | 2 | 2 | 2 |
| | 10 | 4 | 5 | 4 | 4 |

Beispiel 8 Herbizidwirkung für Wasserreis (paddy)

Die Wasserunkräuter Echinochloa crus galli und Monocharia vag. werden in Plastikbechern (60 $cm^2$ Oberfläche, 500 ml Volumen) ausgesät. Nach der Saat wird bis zur Erdoberfläche mit Wasser aufgefüllt. 3 Tage nach der Saat wird der Wasserspiegel bis leicht über die Erdoberfläche erhöht (3-5 mm). Die Applikation erfolgt 3 Tage nach der Saat mit einer wässrigen Emulsion der Prüfsubstanzen durch eine Spritzung auf die Gefässe. Die Dosis entspricht einer Wirkstoffmenge von 4 kg AS pro Hektar (Spritzbrühmenge = 550 l/ha. Die Pflanzenbecher werden dann im Gewächshaus unter optimalen Wachstumsbedingungen für die Reisunkräuter aufgestellt, d.h. bei 25°-30°C und hoher Luftfeuchtigkeit. Die Auswertung der Versuche findet 3 Wochen nach Applikation statt.

Die geprüften Verbindungen der Tabelle 1-6 zeigten in diesem Versuch gute Wirkung.

Beispiel 9: Wuchshemmung bei tropischen Bodenbedecker-Leguminosen (cover crops).

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70 % relativer Luftfeuchtigkeit und 6000 lux Kunstlicht, pro Tag 14 Stunden, bei Temperaturen von 27° bei Tag und 21°C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der Tabellen 1-6 in Aufwandmengen von 50-3000 g/ha behandelten Pflanzen eine deutliche Reduktion des Neuzuwachses (weniger als 20 % des Neuzuwachses bei unbehandelten Kontrollpflanzen), ohne dass dabei die Versuchspflanzen geschädigt wurden.

Beispiel 10: Wuchsregulierung an Sojabohnen

In Kunststoffbehältern mit einem Erde-Torf-Sandgemisch im Verhältnis 6:3:1 werden Sojabohnen der Sorte "Hark" angesät und in eine Klimakammer gegeben. Durch optimale Temperaturwahl, Beleuchtung, Düngerzugabe und Bewässerung entwickeln sich die Pflanzen nach ca. 5 Wochen bis zum 5-6 Trifolia-Blattstadium. Zu diesem Zeitpunkt werden die Pflanzen mit der wässrigen Brühe eines Wirkstoffs der Formel I bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten Kontrollpflanzen bewirken die erfindungsgemässen Wirkstoffe der Tabellen 1-6 eine merklicher Erhöhung der Anzahl und des Gewichts der Schoten im Haupttrieb.

Beispiel 11: Wuchshemmung bei Getreide

In Kunststofftöpfen mit sterilisierter Erde werden in Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Tabellen 1-6 besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleich zu unbehandelten Kontrollen eine Verringerung des Neuzuwachses (60-90 % der Kontrolle), sowie teilweise eine Zunahme der Stengeldurchmesser auf.

Beispiel 12: Wuchshemmung bei Gräsern

In Kunststoffschalen mit Erde-Torf-Sand-Gemisch (6:3:1) werden die Gräser Lolium perenne, Poa pratensis, Festuca ovina, Dactylis glomerate und Cynodon dactylon im Gewächshaus angesät und nach Bedarf bewässert. Die aufgelaufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Tabellen 1-6 besprüht. Die Wirkstoffmenge beträgt umgerechnet bis zu 500 g Aktivsubstanz pro Hektar. 21 Tage nach Applikation wird das Wachstum der Gräser beurteilt.

Die geprüften Verbindungen bewirken eine Reduzierung des Neuzuwachses von um 10-30 % im Vergleich zur unbehandelten Kontrolle.

Beispiel 13: Desiccations- und Defoliationswirkung

In einem Gewächshaus wurden Baumwollpflanzen der Sorte Deltapine in Tontöpfen angezogen. Nach abgeschlossener Kapselbindung wurden sie mit wässerigen Zubereitungen eines Wirkstoffes in einer Aufwandmenge, die 1,2, 0,6 und 0,3 kg/ha im Feld entspräche, gespritzt. Unbehandelte Pflanzen wurden als Kontrolle belassen. Die Auswertung des Versuches erfolgte 3, 7 und 14 Tage nach Applikation der Wirksubstanz durch Bestimmen des Grades an Defoliation (% abgefallene Blätter) und an Desiccation (% Austrocknung der an der Pflanze verbleibenden Blätter).

In diesem Versuch beliessen die geprüften Verbindungen der Tabellen 1-6 bei Aufwandmengen von 0,6 und 1,2 kg/ha nach 7 Tagen bloss noch wenige vertrocknete Blätter auf den Pflanzen (> 80 % Blattfall und Austrocknung).

0213079

Patentansprüche

1. Thiazolylalkyl-, Thienylalkylester von α-Imidazolinon-nicotin- und -benzoesäuren der Formel I

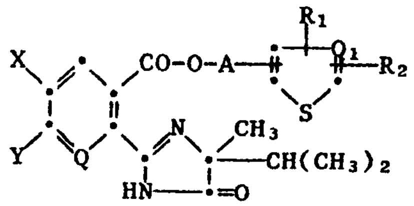

(I)

worin

A eine geradkettige oder verzweigte $C_1$-$C_6$-Alkylenbrücke,

Q und $Q_1$ unabhängig voneinander je Stickstoff oder die Methingruppe,

$R_1$ und $R_2$ unabhängig voneinander je Wasserstoff oder $C_1$-$C_4$-Alkyl,

X und Y unabhängig voneinander je Wasserstoff, $C_1$-$C_4$-Alkyl, $C_1$-$C_4$-Alkoxy, $C_3$-$C_4$-Alkenyl, $C_1$-$C_4$-Halogenalkyl, Halogen oder zusammen den Butadienrest bedeuten.

2. 2-Thiazolylalkyl-α-imidazolinon-nicotinsäureester gemäss Anspruch 1 der Formel Ia

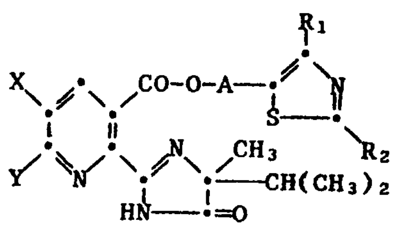

(Ia)

worin $R_1$, $R_2$, X und Y je Wasserstoff oder $C_1$-$C_4$-Alkyl bedeuten und A die im Anspruch 1 gegebene Bedeutung hat.

3. 2-(5-Isopropyl-5-methyl-4-oxo-4,5-dihydro-imidazolin-2-yl)-nicotinsäure-(4-methyl-thiazol-5-yl-äthyl)ester gemäss Anspruch 1.

4. 5-Methyl-2-(5-isopropyl-5-methyl-4-oxo-4,5-dihydro-imidazolin-2-yl)-nicotinsäure-(4-methyl-thiazol-5-yläthyl)ester gemäss Anspruch 1.

5. 5-n-Propyl-2-(5-isopropyl-5-methyl-4-oxo-4,5-dihydro-imidazolin-2-yl)-nicotinsäure-(4-methyl-thiazol-5-yläthyl)ester gemäss Anspruch 1.

6. 5-Isopropyl-2-(5-isopropyl-5-methyl-4-oxo-4,5-dihydroimidazolin-2-yl)nicotinsäure-(4-methyl-thiazol-5-yl-äthyl)ester gemäss Anspruch 1.

7. 2-Thienylalkyl-α-imidazolinon-nicotinsäureester gemäss Anspruch 1 der Formel Ib

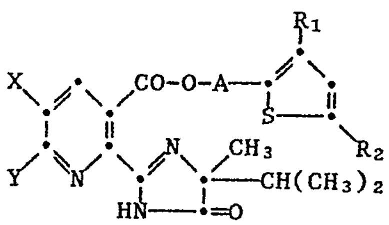

(Ib)

worin $R_1$, $R_2$, X und Y je Wasserstoff oder $C_1$-$C_4$-Alkyl bedeuten und A die im Anspruch 1 gegebene Bedeutung hat.

8. 2-(5-Isopropyl-5-methyl-4-oxo-4,5-dihydro-imidazolin-2-yl)-nicotinsäure-thiophen-2-yl-äthylester gemäss Anspruch 1.

9. 5-Methyl-2-(5-isopropyl-5-methyl-4-oxo-4,5-dihydro-imidazolin-2-yl)-nicotinsäure-(4-methyl-thiophen-2-yl-äthyl)ester gemäss Anspruch 1.

10. 5-Aethyl-2-(5-isopropyl-5-methyl-4-oxo-4,5-dihydro-imidazolin-2-yl)-nicotinsäure-(4-methyl-thiophen-2-yl-äthyl)ester gemäss Anspruch 1.

11. 5-Methyl-2-(5-isopropyl-5-methyl-4-oxo-4,5-dihydro-imidazolin-2-yl)-nicotinsäure-(4-methyl-thiophen-2-yl-methyl)ester gemäss Anspruch 1.

12. 5-Aethyl-2-(5-isopropyl-5-methyl-4-oxo-4,5-dihydro-imidazolin-2-yl)-nicotinsäure-(4-methyl-thiophen-2-yl-methyl)ester gemäss Anspruch 1.

13. 2-Thiazolylalkyl-α-imidazol-benzoesäureester gemäss Anspruch 1 der Formel Ic

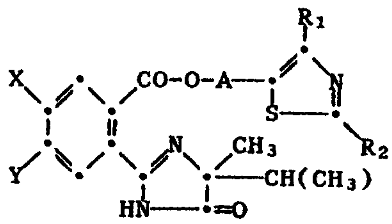

(Ic)

worin $R_1$, $R_2$ und Y Wasserstoff oder $C_1$-$C_4$-Alkyl bedeuten oder X und Y zusammen die Butadien-brücke bilden, während A die im Anspruch 1 gegebene Bedeutung hat.

14. 2-(5-Isopropyl-5-methyl-4-oxo-4,5-dihydro-imidazolin-2-yl)-benzoesäure-(4-methyl-thiadiazol-5-yläthyl)ester gemäss Anspruch 1.

15. 2-Thienylalkyl-α-imidazolinon-benzoesäureester gemäss Anspruch 1 der Formel Id

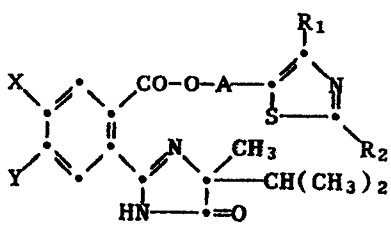

(Id)

worin $R_1$, $R_2$, X und Y Wasserstoff oder $C_1$-$C_4$-Alkyl bedeuten oder X und Y zusammen die Butadienbrücke bilden, während A die im Anspruch 1 gegebene Bedeutung hat.

16. 2-Thiazolalkyl- und 2-Thienylmethylester der α-imidazolinon-chinon-3-carbonsäure gemäss Anspruch 1 der Formel Ig

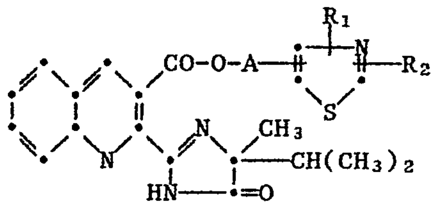

(Ig)

worin $R_1$ und $R_2$ je Wasserstoff oder $C_1$-$C_4$-Alkyl bedeuten und A die im Anspruch 1 gegebene Bedeutung hat.

17. Verfahren zur Herstellung der 2-Thiazolylalkyl- und 2-Thienylalkylester von α-Imidazolinon-nicotin- und -benzoesäuren der Formel I, Anspruch 1, dadurch gekennzeichnet, dass man eine Verbindung der Formel IIa resp. IIb

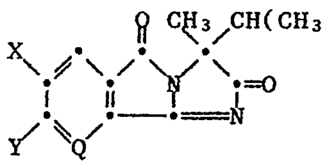

(IIa)

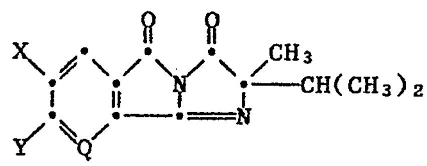

(IIb)

worin Q, X und Y die im Anspruch 1 gegebene Bedeutung haben, in einem inerten organischen Lösungs- oder Verdünnungsmittel in Gegenwart eines basischen Mittels, mit einer Verbindung der Formel III umsetzt,

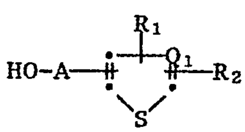

(III)

worin $Q_1$, $R_1$ und $R_2$ die im Anspruch 1 gegebene Bedeutung haben.

18. Ein herbizides und den Pflanzenwachstum regulierendes Mittel, dadurch gekennzeichnet, dass es neben Träger- und/oder anderen Zuschlagstoffen als Wirkstoff 2-Thiazolalkyl- oder 2-Thienylalkylester, einer 2-α-Imidazolinon-nicotin- oder -benzoesäuren gemäss Anspruch 1 enthält.

19. Die Verwendung eines Wirkstoffes gemäss Anspruch 1, oder einen solchen enthaltendes Mittel zur Bekämpfung unerwünschten Pflanzenwachstums.

20. Die Verwendung eines Wirkstoffes gemäss Anspruch 1 oder einem solchen enthaltendes Mittel zur Hemmung des Pflanzenwachstums.

21. Die Verwendung eines Wirkstoffes gemäss Anspruch 1, oder einen solchen enthaltendes Mittel zur Beeinflussung des Pflanzenwachstums zum Zweck der Ertragssteigerung.

22. Verfahren zur selektiven pre- oder postemergenten Bekämpfung von Unkräutern in Nutzpflanzenkulturen, dadurch gekennzeichnet, dass man die Nutzpflanzenkulturen oder deren Anbaufläche mit einer wirksamen Menge einer Imidazolinon-Verbindung der Formel I, Anspruch 1 oder eines eine solche Verbindung enthaltendes Mittel behandelt.

23. Verfahren zur Unterdrückung des Pflanzenwachstums über das 2-Blattstadium hinaus, dadurch gekennzeichnet, dass man die Pflanzen während ihres Wachstums mit einer wirksamen Menge einer Imidazolinon-Verbindung der Formel I Anspruch 1 oder eines einen solchen Wirkstoff enthaltenden Mittels behandelt.

FO 7.5/NU/eg*/cc*